(19) Europäisches Patentamt
European Patent Office
Office européen des brevets

(11) Publication number : 0 348 976 B1

# (12) EUROPEAN PATENT SPECIFICATION

(45) Date of publication of patent specification : **02.09.92 Bulletin 92/36**

(51) Int. Cl.$^5$ : **A61K 7/06**, A61K 47/32

(21) Application number : **89111864.8**

(22) Date of filing : **29.06.89**

## (54) Liquid composition having low-temperature stability.

(30) Priority : **30.06.88 JP 163594/88**

(43) Date of publication of application : **03.01.90 Bulletin 90/01**

(45) Publication of the grant of the patent : **02.09.92 Bulletin 92/36**

(84) Designated Contracting States : **AT BE CH DE ES FR GB GR IT LI LU NL SE**

(56) References cited :
**EP-A- 0 201 342**
**US-A- 4 358 567**

(73) Proprietor : **LION CORPORATION**
**3-7, Honjo 1-chome**
**Sumida-ku Tokyo (JP)**

(72) Inventor : **Nishida, Yuichi**
**No. 38-7, Egota 1-chome Nakano-ku**
**Tokyo (JP)**

(74) Representative : **TER MEER - MÜLLER - STEINMEISTER & PARTNER**
**Mauerkircherstrasse 45**
**W-8000 München 80 (DE)**

**EP 0 348 976 B1**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a liquid composition for cosmetic compositions, skin liniments or the like which comprises a crystalline compound such as an aliphatic acid having a carbon number of 8 to 30 and a salt thereof, an aliphatic alcohol having a carbon number of 8 to 30 and an aliphatic acid derivative having a carbon number of 8 to 30 dissolved in water or an organic solvent, and more specifically, relates to a liquid composition having an excellent low-temperature stability in which the above described crystalline compound is inhibited from crystallizing at a low temperature as efficiently as possible.

There has heretofore been known transparent liquid cosmetic compositions and the like which comprise a crystalline compound such as an aliphatic acid having a carbon number of 8 to 30 and a salt thereof, an aliphatic alcohol having a carbon number of 8 to 30 and an aliphatic acid derivative having a carbon number of 8 to 30 dissolved in water or an organic solvent. However, when these crystalline compounds are dissolved to formulate a liquid product, formulation amount thereof is limited since the crystalline compounds have in general a specific solubility in various solvents, respectively. Moreover even if these crystalline compounds are completely dissolved in a solvent, once the temperature is lowered, turbidity of the solution or precipitation of the crystals may possibly be observed due to temperature-dependency of the solubility. Accordingly, for the purpose of inhibiting turbidity of the solution or precipitation of the crystals at a low temperature, a crystalline compound may be formulated within the solubility limits. However, even under such a condition, a third component to be formulated therewith may sometimes cause co-precipitation phenomenon to generate turbidity of the solution or precipitation of the crystals.

Particularly, in a transparent liquid product, turbidity of solution or precipitation of crystals observed at a low temperature depending on the chemical structure of a substance to be formulated may raise a fatal problem and there is a fear that an effect of the product may be reduced if the crystalline compound is an effective component in the product.

In spite of the above described situation, a liquid product for cosmetic compositions, skin liniments or the like is required, from view of a value as a merchandize, to exhibit a stable quality under any preservation conditions from a lower temperature of around -5°C in winter to a higher temperature of 40°C or higher.

Heretofore, as a method for inhibiting crystallization or precipitation of crystals in a liquid system, there has been proposed, in Japanese Patent Application Kokai Nos. 129096/1986 and 84186/1987, a method in which a fuel oil and a copolymer of ethylene and carboxylic acid vinyl ester are used. Further, employment of a non-ionic active agent having a HLB value of 10 or less as a low-temperature stabilizer for an aliphatic acid or a derivative thereof has been proposed in Japanese Patent Application Kokai No. 15815/1986 and employment of polyvinyl pyrrolidone for inhibiting precipitation of a low solubility pharmaceutical in a gel-stated composition has been proposed in Japanese Patent Application Kokai No. 183615/1983, respectively. However, in these methods, stabilization effects at a low temperature are still unsatisfactory, and particularly in a system in which compounds are dissolved in water or an organic solvent miscible with water cannot be exerted sufficiently. Thus, it has been desired to develop a liquid composition in which a crystalline compound has a higher solubility than the conventional ones even at a low temperature.

Therefore an object of the present invention is to provide a liquid composition comprising a crystalline compound with an excellent low-temperature stability in which turbidity or precipitation of the crystalline compounds are hardly observed even when stored at a low temperature over a long period.

The present inventor has studied intensively in order to accomplish the above described object, and as a result, found that, by formulating at least one kind of amphoteric polymers containing a structural unit represented by the following formula [I]:

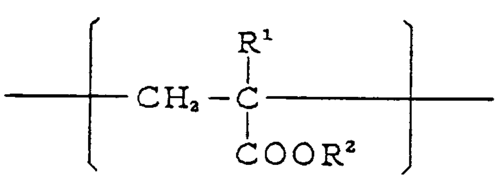

··· [ I ]

wherein $R^1$ represents a hydrogen atom or a methyl group, $R^2$ represents an alkyl group having a carbon number of 8 to 20 or a cycloalkyl group having a carbon number of 5 to 7,

into a liquid composition comprising a crystalline compound such as an aliphatic acid having a carbon number of 8 to 30 and a salt thereof, an aliphatic alcohol having carbon number of 8 to 30 and an aliphatic acid derivative having a carbon number of 8 to 30 dissolved in water or an organic solvent, there can be obtained a liquid compositions having an excellent low-temperature stability in which these crystalline compounds are inhibited

from crystallizing at a low temperature to exhibit excellent improving effects in solubility and low-temperature stability, and turbidity of the solution of precipitation of the crystals are hardly observed even when stored at a low temperature of around -5°C over a long period.

Further, it has been found that the liquid composition preferably contains as the amphoteric polymer, in addition to the above described structural unit of the formula [I], a structural unit represented by the following formula [II]:

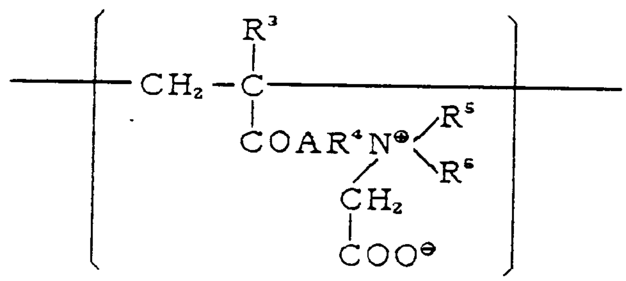

··· [II]

wherein $R^3$ is a hydrogen atom or methyl group, $R^4$ is an alkylene group having a carbon number of 1 to 4, $R^5$ and $R^6$ are a hydrogen atom or an alkyl group having a carbon number of 1 to 4, respectively, and A is an oxygen atom or NH group,

and that the above described effects can be more efficiently exhibited by additing a non-ionic surfactant having a HLB value of 10 or less, in addition to the above described amphoteric polymers.

As examples utilizing the amphoteric polymers to be used in the present invention as cosmetic materials, there can be mentioned Japanese Patent Application Kokai No. 92809/1981 and the like. However, these polymers are all used as a resin for hair conditioners and any suggestions as to stabilizing effect of the crystalline compounds are not disclosed.

The liquid composition according to the present invention contains an amphoteric polymer as an agent for inhibiting crystallization of the crystalline compound as described above.

The amphoteric polymer to be used in the present invention has a structural unit represented by the following formula [I]:

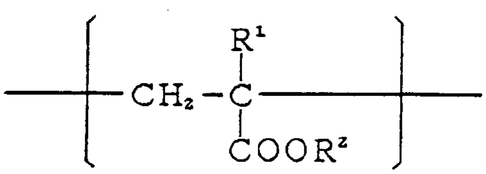

··· [I]

wherein $R^1$ represents a hydrogen atom or a methyl group, $R^2$ represents an alkyl group having a carbon number of 8 to 20, more preferably 12 to 16 or a cycloalkyl group having a carbon number of 5 to 7.

In this case, as the amphoteric polymer, there may be suitably used a polymer in which, as a substituent $R^2$ in the structural unit of the formula [I], an alkyl group having a carbon number of 12 to 16 and a cycloalkyl group having a carbon number of 5 to 7 are contained as a mixture, particularly, an alkyl group having a carbon number of 12 to 16 and a cycloalkyl group having a carbon number of 5 to 7 are contained at a mixing ratio of 5:95 to 95:5, preferably 10:90 to 90:10 in terms of weight ratio of monomer.

As a monomer giving the structural unit of the above mentioned formula [I], there may be exemplified an ester of acrylic acid or methacrylic acid, for example, 2-ethylhexylacrylate and -methacrylate, i-tridecanyl acrylate and -methacrylate, pentadecanyl acrylate and -methacrylate, stearyl acrylate and -methacrylate, lauryl acrylate and -methacrylate, cyclohexyl acrylate and -methacrylate. Further, there may be mixed therewith an ester of acrylic acid or methacrylic acid having a lower alkyl group having a carbon number of 7 or less such as isobutyl acrylate or -methacrylate. In this case, the average carbon number of the alkyl groups in $R^2$ of the formula [I] should preferably be 5 or more.

Moreover, the amphoteric polymer having the structural unit of the formula [I] described above may further contain a group or a structural unit derived from a monomer capable of copolymerizing with

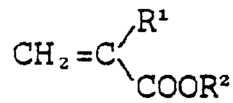

and of giving an ionic property thereon, as exemplified by a structural unit represented by the following formula [II] :

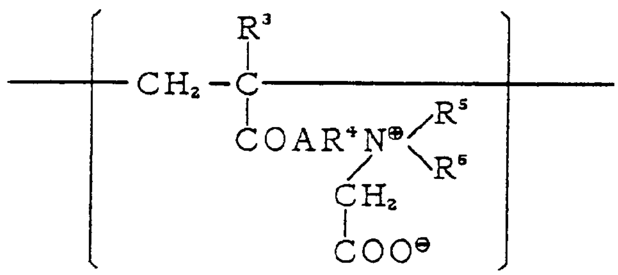

··· [II]

wherein $R^3$ is a hydrogen atom or methyl group, $R^4$ is an alkylene group having a carbon number of 1 to 4, $R^5$ and $R^6$ are a hydrogen atom or an alkyl group having a carbon number of 1 to 4, respectively, and A is an oxygen atom or NH group.

Examples of the structural unit [II] may be betaine dialkylaminoalkyl acrylate, betaine dialkylaminoalkyl methacrylate, betaine dialkylaminoalkyl acrylamide, or betaine dialkylaminoalkyl methacrylamide.

More preferably, the structural unit of the formula [II] described above can be given from a polymeric monomer such as dimethylaminoethyl acrylate and -methacrylate, diethylaminoethyl acrylate and -methacrylate, dimethylaminopropyl acrylate and -methacrylate, dimethylaminoethyl acrylamide and -methacrylamide and diethylaminopropyl acrylamide and -methacrylamide by polymerizing the monomer or monomers and modifying the resulting polymer with a halogenated acetic acid and the like.

Moreover, the amphoteric polymer of the present invention may contain in its molecular a structural unit of the other hydrophilic ethylenic monomers in addition to the structural units of the formula [I] and the formula [II] described above.

As the hydrophilic ethylenic monomer herein, there may be used, for example, N-vinyl pyrrolidone, acrylamide, hydroxyethyl acrylate and -methacrylate, hydroxypropyl acrylate and -methacrylate, ethylene glycol monoacrylate and -monomethacrylate, polyethylene glycol monoacrylate and -monomethacrylate, propylene glycol monoacrylate and -monomethacrylate, polypropylene glycol monoacrylate and -monomethacrylate, acrylonitrile, styrene, chlorostyrene, vinyl toluene, vinyl acetate, vinyl trichlorosilane, methacryloxypropyl trimethoxysilane and the like.

Thus, the amphoteric polymer according to the present invention has in its molecule the structural unit of the formula [I] as an essential structure and at the same time preferably may have the structural unit of the formula [II] and further additionally may contain the structural unit of the above-said hydrophilic ethylenic monomer, and these monomers may be variously selected depending on uses thereof to prepare a monopolymer, dipolymer, terpolymer, tetrapolymer, pentapolymer, etc.

The ratio of the structural unit of the above described formula [I] in the amphoteric polymer is not particularly limited, however, preferably is 5 to 80% (% by weight, the same can be applied hereinafter), particularly 30 to 90% based on the total weight of the polymer. The ratio of the structural unit of the formula [II] is preferably 30 to 90%, particularly 40 to 80%, and the ratio of the structural unit of the above-said hydrophilic ethylenic monomer is preferably 0 to 90%, particularly 40 to 80%.

In the amphoteric polymer, it is preferable that the average molecular weight thereof is 400 to 2,000,000, particularly 7,000 to 150,000 and the percentage of amphoterization is 20 to 100%.

Among the amphoteric polymers described above, there may suitably be used an amphoteric polymer in which $R^2$ is an alkyl group having carbon number of 8 to 20, particularly 12 to 16 or a cycloalkyl group having a carbon number of 5 to 7 and $R^1$, $R^3$, $R^5$ and $R^6$ are a methyl group, respectively, $R^4$ is an ethylene group, A is an oxygen atom in the structural units of the above described formula [I] and the formula [II] and the average molecular weight thereof is 50,000 to 90,000, as exemplified by a mixed alkyl copolymer such as N-methacryloylethyl-N,N-dimethyl ammonium α-N-methylcarboxybetain $C_8$ to $C_{20}$ methacrylate.

The amphoteric polymer can be prepared according to the same manner as in the conventional polymer and can be easily obtained by using as a solvent a hydrophilic solvent, for example, an aliphatic alcohol such

as a monoalcohol, e.g. methanol, ethanol and isopropanol and a polyalcohol, e.g. ethylene glycol, ethylene glycol ethyl ether or -butyl ether (among these, an alcohol having 1 to 4 carbon atoms, is preferable) or esters such as methyl acetate, dioxane, dimethylformamide, etc., and copolymerizing the above described monomers in these solvents, and then subjecting the resulting polymer to reaction with a halogenated acetic acid salt represented by the following formula:

$$XCH_2COOM$$

wherein X is a halogen atom such as chlorine, bromine, iodine and the like, M is a salt with an alkaline metal such as sodium, potassium and the like,
in a hydrophilic solvent.

As the amphoteric polymer described above, for example, YUKAFORMER® (commercially available from Mitsubishi Yuka K. K.) is suitably employed.

In the composition of the present invention, the formulation amount of the amphoteric polymer, although it can suitably be regulated depending on the formulation amount of the crystalline compounds, is preferably 0.001 to 10%, particularly 0.01 to 1% based on the total weight of the composition.

In the composition of the present invention, it is preferable to formulate a non-ionic surfactant having a paste or liquid form at -5°C and a HLB value of 10 or less, together with the above described amphoteric polymers.

The paste or liquid non-ionic surfactant may include sorbitan aliphatic acid ester, polyoxyethylene sorbitan aliphatic acid ester, polyoxyethylene sorbitol aliphatic acid ester, polyoxyethylene hydrogenated castor oil, polyoxyethylene hydrogenated castor oil aliphatic acid ester, polyglycerine aliphatic acid ester, branch chained fatty acid ester, branch chained fatty alcohol ester N-long chained acyl-L-glutamic acid higher alcohol diester, N-long chained acyl-L-glutamic acid polyoxyethylene higher alcohol ether diester and the like. In these non-ionic surfactants, HLB value can usually be regulated by moles of ethylene oxide or propylene oxide added or a length of carbon chain of a lipophilic group. If a non-ionic surfactant having a HLB value exceeding 10 is used, the low-temperature stability of the composition is sometimes not improved and thus the HLB value of the non-ionic surfactant is desirably 10 or less.

Preferred are a mixture of mono, di and tri esters of lauryl sorbitan ester, octyl-dodecyl lactate represented by the following formula:

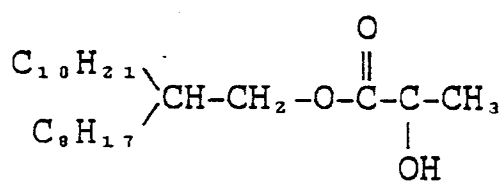

neopentyl glycol dioctanate represented by the following formula:

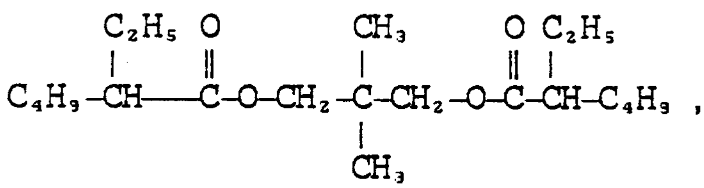

and succynyl polypropylen glycol oligo ester represented by the following formula:

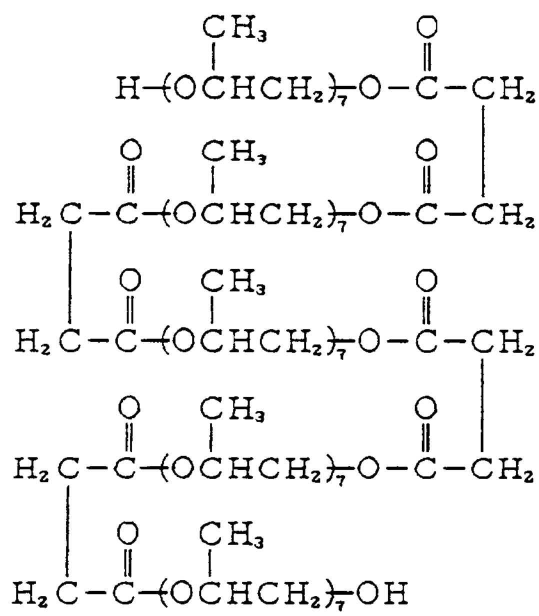

The formulation amount of the non-ionic surfactant is usually about 0.1 to 10% and it is particularly preferable to be 0.5 to 8%.

The liquid composition of the present invention is one in which the above described amphoteric polymers and more preferably a non-ionic surfactant having a HLB value of 10 or less are formulated to a crystalline compound dissolved in a solvent, and by such a formulation, a crystalline compound, particularly a compound having a temperature gradient in solubility in a solvent, such as an aliphatic acid having a carbon number of 8 to 30 and a salt thereof, an aliphatic alcohol having a carbon number of 8 to 30 and an aliphatic acid derivative having a carbon number of 8 to 30 can be inhibited from crystallizing at a low temperature as efficiently as possible.

As the aliphatic acid, there may be mentioned, for example, octanoic acid, nonanoic acid, decanoic acid, undecanoic acid, dodecanoic acid, tetradecanoic acid, pentadecanoic acid, hexadecanoic acid, heptadecanoic acid, octadecanoic acid, nonadecaconic acid, eicosanoic acid, heneicosanoic acid, docosanoic acid, tricosanoic acid, tetracosanoic acid, pentacosanoic acid and the like. Salts thereof may include an alkaline metal salt thereof, an ammonium salt thereof, an organic amine salt thereof and the like.

As the aliphatic alcohol, there may be exemplified decanol, undecanol, dodecanol, tridecanol, tetradecanol, pentadecanol, hexadecanol, heptadecanol, octadecanol, nonadecanol, eicosanol, heneicosanol, docosanol, tricosanol, tetracosanol, pentacosanol and the like.

As the aliphatic acid derivative, derivatives from the above described aliphatic acids or aliphatic alcohols are preferable, as exemplified by the following crystalline compounds (a) to (j):

(a) Glycerine ester represented by the following formulas:

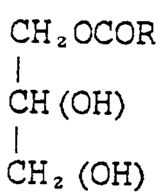 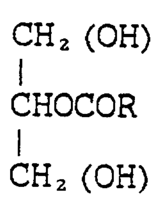 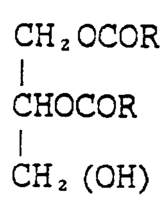 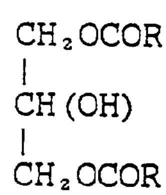 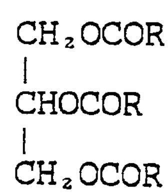

wherein, R is an aliphatic acid residue having a carbon number of 8 to 30.

(b) Ester represented by the following formula:

RCOOR′

wherein R′ represents a monohydroxy or dihydroxy alcohol residue such as methanol, ethanol and the like, a polyoxyethylene residue, a sorbitan residue or a sucrose residue, and R is the same meaning as above.

(c) Amide represented by the following formulas:

RCONR"R"' 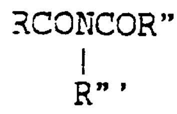 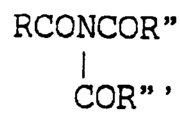

wherein R" and R''' are a hydrogen atom or an organic group, such as a residue of amino acid, an alkyl group, an alkenyl group or a phenyl group, respectively, and R is the same meaning as above.

(d) Dibasic acid represented by the following formula and a salt thereof:

MOOCRCOOM

wherein M is an alkali metal, an alkaline earth metal, ammonium or an organic amine, and R is the same meaning as above.

(e) Sterol ester represented by the following formula:

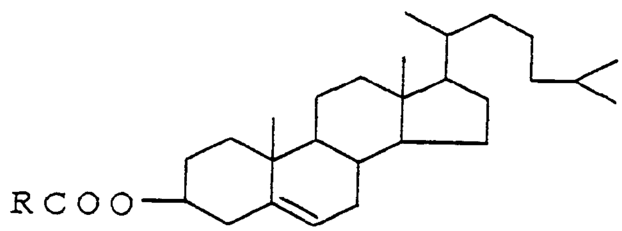

wherein R is the same meaning as above.

(f) Phospholipid represented by the following formula:

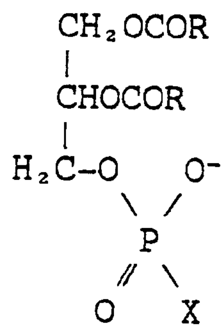

wherein X represents a choline residue, an ethanolamine residue, a serine residue or an inositol residue. A phospholipid is phosphatidylcoline when X is a choline residue, phosphatidylethanolamine when an ethanolamine residue, phosphatidylserine when a serine residue, and phosphatidylinositol when an inositol residue. R is the same meaning as above.

(g) Phosphatidic acid represented by the following formula:

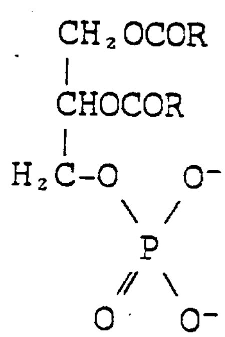

wherein R is the same meaning as above.

(h) Sphingolipid represented by the following formula:

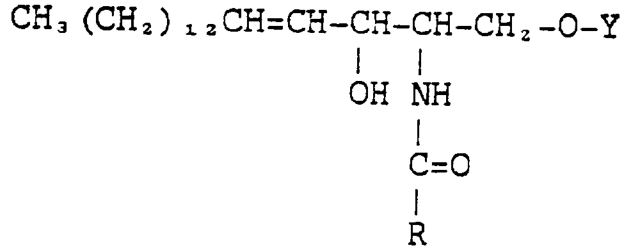

wherein Y represents a sugar residue, a phospholic acid residue or an amine base residue such as choline or ethanolamine, and R is the same meaning as above.

(i) Ester represented by the following formula:

$$R_a\text{-O-}R_b$$

wherein $R_a$ is an aliphatic alcohol residue having an alkyl group having a carbon number of 8 to 30, $R_b$ represents an aliphatic acid residue (preferably ones having a chain length of $C_2$ to $C_{24}$); an organic acid such as succinic acid, oxalic acid, fumaric acid, lactic acid, pyruvic acid, malic acid, oxaloacetic acid; an inorganic acid residue such as phospholic acid.

(j) Ether represented by the following formula:

$$R_a\text{-O-}R_c$$

wherein $R_c$ represents an monohydroxy alcohol residue, preferably ones having a chain length of $C_2$ to $C_{24}$; a polyhydroxy alcohol such as glycerine, polygrycerine, ethylene glycol, propylene glycol and butanediol; a sugar residue such as glucose, ribose, galactose, arabinose, mannose, xylose, sorbitol and mannitol, and $R_a$ is the same meaning as above.

As the crystalline compound other than the compounds described above, vitamins such as biotine, riboflavin, ascorbic acid and cyanocobalamin; amino acids such as glutamic acid, lisine and cysteine; electrolytes such as glutathione, quinones and perchloric acid tetrabutylammonium can be also used.

As the crystalline compound, a single kind or two or more kinds of the compounds described above can be used. Among the crystalline compounds, derivatives of an aliphatic acid or a polyhydroxy alcohol of an aliphatic alcohol, particularly, glycerine aliphatic acid ester, sorbitol aliphatic acid ester, sucrose aliphatic acid ester are preferable.

The solvent to be used in the liquid composition of the present invention is not particularly limited, and either water or an organic solvent can be used so long as it is a solvent capable of dissolving the amphoteric polymer and the crystalline compound therein. As the organic solvent, a hydrophilic solvent or a lipophilic solvent can be used, however, particularly a nonaqueous solvent having a water content of 20% or less, particularly 5% or less can preferably be used. As specific examples for such a nonaqueous solvent, methyl alcohol, ethyl alcohol, propyl alcohol, isopropyl alcohol, glycerine, propylene glycol polyethylene glycol, n-paraffin, benzene, hexane, chloroform and the like can be exemplified and particularly, a lower alcohol and a polyhydroxy alcohol are preferable, and particularly ethyl alcohol which is highly safe can be desirably used when the present composition is used for cosmetic compositions or skin liniments.

The formulation amount of the crystalline compound described above is preferably set within a range between an amount corresponding to a solubility at -20°C or more and an amount corresponding to a solubility at 30°C or less. In general, the formulation amount of the crystalline compound, although not particularly limited, is preferably 0.01 to 50%, particularly 1 to 5% based on the total weight of the composition.

In the liquid composition of the present invention, other components than described above may optionally be formulated depending on use objects.

For example, a polyhydroxy alcohol, surfactants other than the liquid or paste non-ionic surfactants having a HLB value of 10 or less, pharmaceutical components such as fats and oils, hormones, vasodilators, amino acids, anti-inflammatory agents, skin function sthenic agents, keratin improving agents can be formulated therewith to be utilized as various cosmetic compositions such as hair cosmetic agents, (e.g. hair nourishing agent, hairtonic, hair liquid, hair spray); cosmetic agents for skin care; and nail agents, or as pharmaceuticals such as skin liniments. Besides, dye materials paraffins, petroleums, lime or the like can be formulated therewith to be widely utilized as industrial products such as paints, fuel oils and the like.

In the liquid composition of the present invention, the amphoteric polymer containing the structural unit of the above described formula [I] inhibits the crystalline compound from crystallizing at a low temperature, thereby to exhibit an excellent effect for improving a solubility and an excellent effect for improving a low-temperature stability, so that turbidity of the solution or precipitation of the crystals are hardly observed even if stored at a low temperature of around -5°C over a long period of time to impart it an excellent low-temperature stability.

The present invention will be described below more specifically by referring to Examples.

[Example 1]

Samples having a formulation as shown in Tables 1 to 4 were prepared. Stabilized days and a low-temperature stability were evaluated thereon according to a method as shown below, thereby to determine an effect for inhibiting crystallization, respectively.

The results obtained are also shown in Tables 1 to 4.

⟨Method for evaluating stabilized days and a low-temperature stability⟩

About 50 g of samples to be tested were taken into a transparent glass bottle, stored in a thermostatic chamber maintained at -5°C, and a number of days obtained by subtracting one day from a number of days required for crystals to be firstly observed was designated as "stabilized days". The results are shown as a number of days obtained by rounding an average number of days from three times testing results to the nearest whole number.

The low-temperature stabilities were evaluated as O when stabilized days are 28 days or more, Δ when 14 to 27 days and X when 13 days or less.

Table 1

| | | | | No. 1 | No. 2 | No. 3 | No. 4 | No. 5 |
|---|---|---|---|---|---|---|---|---|
| Components (% by weight) | Crystalline compound: Palmitic acid monoglyceride (Monoglyceride content: 95%) | | | 3 | 3 | 3 | 3 | 3 |
| | Copolymer of N-methacryloyl ethyl-N, N-dimethyl ammonium α-N-methyl-carboxybetain alkyl methacrylate (Percentage of amphoterization: 60) (Average molecular weight: 70, 000) | Carbon number | 4*[1] | 0. 1 | | | | |
| | | | 6*[2] | | 0. 1 | | | |
| | | | 12*[3] | | | 0. 1 | | |
| | | | 13*[4] | | | | 0. 1 | |
| | | | Mix*[5] | | | | | 0. 1 |
| | Ethyl alcohol/Water = 99. 5/0. 5 | | | ←91. 9→ | | | | |
| | Sorbitan monolaurate | | | 5 | 5 | 5 | 5 | 5 |
| | Total | | | ←100. 0→ | | | | |
| Evaluation | Stabilized days (day) | | | 15 | 45 | 50 | 46 | 65 |
| | Low-temperature stability | | | Δ | O | O | O | O |
| | | | | Invention | | | | |

*[1]: Iso-$C_4$ *[2]: Cyclo-$C_6$ *[3]*[4]: Straight $C_{12}$ and $C_{13}$, respectively. *[5]: Mixed system of iso-$C_4$, cyclo-$C_6$ and straight $C_{12}$ and $C_{13}$ (Mixing weight ratio: 36:16:12:26, Average carbon number: 7).

Table 2 (1)

| | | | | No. 6 | No. 7 | No. 8 | No. 9 | No. 10 | No. 11 | No. 12 |
|---|---|---|---|---|---|---|---|---|---|---|
| Components (% by weight) | Crystalline compound: Palmitic acid monoglyceride (Monoglyceride content: 95%) | | | 3 | 3 | 3 | 3 | 3 | 3 | 3 |
| | Copolymer of N-methacryloyl ethyl-N, N-dimethylammonium α N-methylcarboxybetain alkyl methacrylate *6 (Average molecular weight: 90, 000) | amphoterization | 0% | 0..1 | | | | | | 0. 1 |
| | | | 30% | | 0. 1 | | | | | |
| | | | 50% | | | 0. 1 | | | | |
| | | | 60% | | | | 0. 1 | | | |
| | | | 70% | | | | | 0. 1 | | |
| | | | 80% | | | | | | 0. 1 | |
| | Ethyl alcohol/Water = 99. 5/0. 5 | | | ←96. 9→ | | | | | | 91. 9 |
| | Sorbitan monolaurate | | | - | - | - | - | - | - | 5 |
| | Total | | | ←100. 0→ | | | | | | |
| Evaluation | Stabilized days (day) | | | 7 | 28 | 30 | 31 | 32 | 31 | 21 |
| | Low-temperature stability | | | X | O | O | O | O | O | Δ |
| | | | | Comparative | The invention | | | | | Comparative |

Table 2 (2)

| | | | | No. | | | | | | |
|---|---|---|---|---|---|---|---|---|---|---|
| | | | | 13 | 14 | 15 | 16 | 17 | 18 | 19 |
| Components (% by weight) | Crystalline compound: Palmitic acid monoglyceride (Monoglyceride content: 95%) | | | 3 | 3 | 3 | 3 | 3 | 3 | 3 |
| | Copolymer of N-methacryloyl ethyl-N, N-dimethylammonium α-N-methylcarboxybetain alkyl methacrylate *[6] (Average molecular weight: 90, 000) | amphoterization | 0% | | | | | | | |
| | | | 30% | 0. 1 | | | | | | |
| | | | 50% | | 0. 1 | | | | | |
| | | | 60% | | | 0. 1 | | | | |
| | | | 70% | | | | 0. 1 | | | |
| | | | 80% | | | | | 0. 1 | | |
| | Ethyl alcohol/Water = 99. 5/0. 5 | | | ←91. 9→ | | | | | 92 | 97 |
| | Sorbitan monolaurate | | | 5 | 5 | 5 | 5 | 5 | 5 | – |
| | Total | | | ←100. 0→ | | | | | | |
| Evaluation | Stabilized days (day) | | | 42 | 40 | 41 | 40 | 31 | 21 | 7 |
| | Low-temperature stability | | | O | O | O | O | O | Δ | X |
| | | | | The invention | | | | | Comparative | |

*[6]: Kind of alkyl: Mixed system of iso-$C_4$ and $C_{12}$ (Mixing weight ratio: 10:90, Average carbon number: 11)

Table 3 (1)

| | | | No. | | | | | |
|---|---|---|---|---|---|---|---|---|
| | | | 20 | 21 | 22 | 23 | 24 | 25 |
| Components (% by weight) | Copolymer of N-methacryloyl ethyl-N, N-dimethylammonium α-N-methylcarboxybetain alkyl methacrylate *7 (Average molecular weight: 90,000) (Percentage of amphoterization: 65) | | 0.5 | 0.5 | 0.5 | 0.5 | 0.5 | 0.5 |
| | Crystalline compound | Octoic acid glyceride | 1 | | | | | |
| | | Undecanoic acid glyceride | | 1 | | | | |
| | | Pentadecanoic acid glyceride | | | 1 | | | |
| | | Heptadecanoic acid glyceride | | | | 1 | | |
| | | Pentacosanoic acid glyceride | | | | | 1 | |
| | | Hexacosanoic acid glyceride | | | | | | 1 |
| | Ethyl alcohol | | ←91.5→ | | | | | |
| | POE (5) hardened castor oil | | 7 | 7 | 7 | 7 | 7 | 7 |
| | Total | | ←100.0→ | | | | | |
| Evaluation | Stabilized days (day) | | 56 | 53 | 49 | 46 | 40 | 28 |
| | Low-temperature stability | | ○ | ○ | ○ | ○ | ○ | ○ |
| | | | The invention | | | | | |

Table 3 (2)

| | | | No. | | | | | | | | |
|---|---|---|---|---|---|---|---|---|---|---|---|
| | | | 26 | 27 | 28 | 29 | 30 | 31 | 32 | 33 | 34 |
| Components (% by weight) | Copolymer of N-methacryloyl ethyl-N, N-dimethylammonium α-N-methylcarboxybetain alkyl methacrylate *[7] (Average molecular weight: 90, 000) (Percentage of amphoterization: 65) | | – | – | – | – | – | – | 0. 5 | 0. 5 | 0. 5 |
| | Crystalline compound | Octoic acid glyceride | 1 | | | | | | 1 | | |
| | | Undecanoic acid glyceride | | 1 | | | | | | | |
| | | Pentadecanoic acid glyceride | | | 1 | | | | | 1 | |
| | | Heptadecanoic acid glyceride | | | | 1 | | | | | 1 |
| | | Pentacosanoic acid glyceride | | | | | 1 | | | | |
| | | Hexacosanoic acid glyceride | | | | | | 1 | | | |
| | Ethyl alcohol | | ←92. 0→ | | | | | | ←98. 5→ | | |
| | POE (5) hardened castor oil | | 7 | 7 | 7 | 7 | 7 | 7 | – | – | – |
| | Total | | ←100. 0→ | | | | | | | | |
| Evaluation | Stabilized days (day) | | 7 | 8 | 7 | 6 | 4 | 3 | 32 | 32 | 30 |
| | Low-temperature stability | | X | X | X | X | X | X | O | O | O |
| | | | Comparative | | | | | | The invention | | |

*[7]: Mixed system of iso-$C_4$, cyclo-$C_6$, straight $C_{12}$ and $C_{13}$.
(Mixing weight ratio: 5:45:25:25, Average carbon number: 9)

Table 4

| | | | | No. | | | | | | | |
|---|---|---|---|---|---|---|---|---|---|---|---|
| | | | | 35 | 36 | 37 | 38 | 39 | 40 | 41 | 42 |
| Components (% by weight) | Copolymer of N-methacryloyl ethyl-N, N-dimethylammonium α-N-methylcarboxybetain alkyl methacrylate *[a] (Average molecular weight: 80, 000) | | | 0. 2 | 0. 2 | 0. 2 | 0. 2 | 0. 2 | 0. 2 | 0. 2 | 0. 2 |
| | Non-ionic surfactant (HLB) | Diglycerine diisostearate | (1. 5) | 5 | | | | | | | |
| | | Propylene glycol monolaurate | (4) | | 5 | | | | | | |
| | | Dioctyldodecyl lauroylglutamate | (<5) | | | 5 | | | | | |
| | | Sorbitan monooleate | (4. 3) | | | | 5 | | | | |
| | | Polygclycerine monoisostearate | (7) | | | | | 5 | | | |
| | | POE(5) cholesteryl ether | (9. 5) | | | | | | 5 | | |
| | | POE(6) sodium alkyl ether acetate | (6) | | | | | | | 5 | |
| | | POE(4) sodium lauryl ether phosphate | (1. 3) | | | | | | | | 5 |
| | Ethyl alcohol | | | 90. 8 | | | | | | | |
| | Crystalline compound: Pentadecanoic acid glyceride (Monoglyceride content: 95%) | | | 4 | 4 | 4 | 4 | 4 | 4 | 4 | 4 |
| | Total | | | 100. 0 | | | | | | | |
| Evaluation | Stabilized days (day) | | | 40 | 38 | 42 | 50 | 42 | 33 | 48 | 50 |
| | Low-temperature stability | | | O | O | O | O | O | O | O | O |
| | | | | The invention | | | | | | | |

*[a]: Mixed system of iso-$C_4$, cyclo-$C_6$, straight $C_{12}$ and $C_{13}$.
(Mixing weight ratio: 5:5:40:50, Average carbon number: 12)

From the results in Tables 1 to 4, it has been confirmed that samples containing the amphoteric polymer and the crystalline compound according to the present invention are excellent in the low-temperature stability, and samples containing a liquid or paste non-ionic surfactant having a HLB value of 10 or less formulated therein together with the amphoteric polymer and the crystalline compound is able to maintain well an excellent low-temperature stability.

[Example 2] Hair nourisher

| | |
|---|---|
| Pentadecanoic acid glyceride | 3% |
| Acetic acid dl-α-tocopherol | 0.1 |
| Sorbitan monolaurate | 4 |
| Dicaprylic acid pyridoxine | 0.1 |
| Safflower oil | 0.2 |
| Succinic acid | 0.2 |
| Paste extracted from Japanese chiretta | 0.1 |
| Biotine | 0.001 |
| YUKAFORMER® 205S (manufactured by Mitsubishi Yuka K.K.) | 0.1 |
| Hinokitiol | 0.05 |
| Perfume | 0.3 |
| 99.5% Ethanol | balance |
| Total | 100.0% |

[Example 3] Hair spray

(Undiluted solution)

| | |
|---|---|
| 1-Hexacosene | 1% |
| YUKAFORMER® 201 (manufactured by Mitsubishi Yuka K. K.) | 1 |
| Citric acid | 0. 3 |
| ℓ-Mentol | 0. 1 |
| Sorbitan monooleate | 0. 8 |
| Perfume | 0. 5 |
| Ethanol | balance |
| Total | 100. 0% |

(Diluted solution for filling)

| | |
|---|---|
| Undiluted solution described above | 35% |
| Carbon dioxide gas | 40 |
| LPG | 15 |
| Nitrogen gas | 5 |
| Total | 100% |

[Example 4] Hair lotion

| | |
|---|---|
| Dilead sulfophenylate | 1. 0% |
| Myristyl stearate | 0. 5 |
| Aloe ECW | 0. 5 |
| YUKAFORMER® 202 (manufactured by Mitsubishi Yuka K. K.) | 0. 2 |
| POE (5) hardened castor oil | 3. 0 |

| | |
|---|---|
| Perfume | 0.5 |
| Ethyl alcohol | balance |
| Total | 100.0% |

The compositions in Examples 2 to 4 are all excellent in a low-temperature stability and exhibit an effect for inhibiting crystallization.

## Claims

1. A liquid composition containing a crystalline compound dissolved in a solvent, which comprises at least one kind of amphoteric polymers containing a structural unit represented by the following formula [I]:

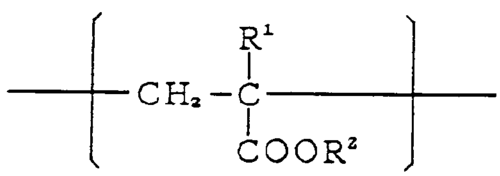

… [ I ]

wherein $R^1$ is a hydrogen atom or a methyl group, $R^2$ is an alkyl group having a carbon number of 8 to 20 or a cycloalkyl group having a carbon number of 5 to 7,
formulated in said composition.

2. The composition according to Claim 1, wherein said amphoteric polymer contains a structural unit represented by said formula [I] and a structural unit represented by the following formula [II]:

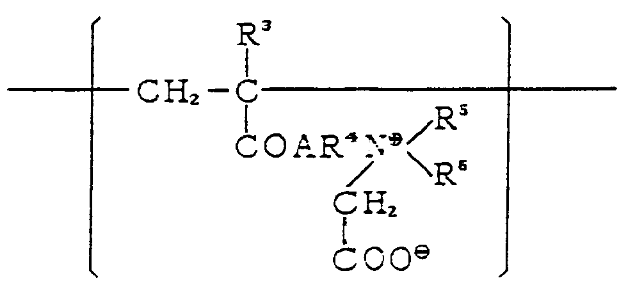

… [ II ]

wherein $R^3$ is a hydrogen atom or methyl group, $R^4$ is an alkylene group having a carbon number of 1 to 4, $R^5$ and $R^6$ are a hydrogen atom or an alkyl group having a carbon number of 1 to 4, respectively, and A is an oxygen atom or NH group.

3. The composition according to Claim 2, wherein said amphoteric polymer contains 5 to 80% by weight of the structural unit of the formula [I], and 30 to 90% by weight of the structural unit of the formula [II] and has an average molecular weight of 400 to 2,000,000.

4. The composition according to Claim 1, wherein said amphoteric polymer contains a structural unit represented by the following formula:

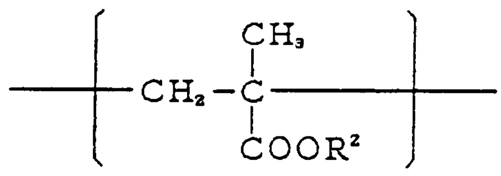

wherein $R^2$ is an alkyl group having a carbon number of 8 to 20 or a cycloalkyl group having a carbon number of 5 to 7, and a structural unit represented by the following formula:

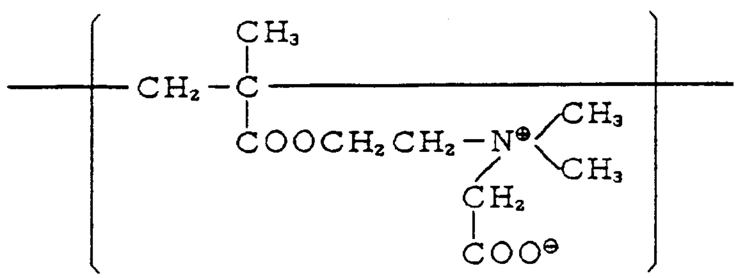

5. The composition according to Claim 1, wherein said amphoteric polymer is blended in an amount of 0.001 to 10% by weight of the composition.

6. The composition according to Claim 1, wherein a non-ionic surfactant having a liquid or paste form at -5°C and a HLB value of 10 or less is formulated therein.

7. The composition according to Claim 6, wherein the non-ionic surfactant having a liquid or paste form at -5°C and a HLB value of 10 or less is formulated in an amount of 0.1 to 10% by weight of the composition.

8. The composition according to Claim 1, wherein said crystalline compound is selected from aliphatic acids having a carbon number of 8 to 30 and salts thereof, aliphatic alcohols having a carbon number of 8 to 30, and aliphatic acid derivatives having a carbon number of 8 to 30.

9. The composition according to Claim 1, wherein said crystalline compound is formulated within a range between an amount corresponding to a solubility at -20°C or more and an amount corresponding to a solubility at 30°C or less.

**Patentansprüche**

1. Flüssige Zusammensetzung, enthaltend eine in einem Lösungsmittel gelöste kristalline Verbindung, umfassend mindestens eine in die Zusammensetzung eingebrachte Art von amphoteren Polymeren, die eine durch die folgende Formel (I) angegebene Struktureinheit enthalten:

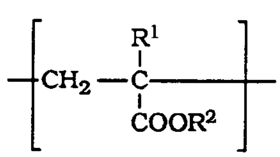 [I]

worin $R^1$ ein Wasserstoffatom oder eine Methylgruppe,
$R^2$ eine Alkylgruppe mit einer Kohlenstoffanzahl von 8 bis 20 oder eine Cycloalkylgruppe mit einer Kohlenstoffansahl von 5 bis 7 bedeuten.

2. Zusammensetzung nach Anspruch 1, wobei das amphotere Polymer eine durch die Formel (I) angegebene Struktureinheit sowie eine durch die folgende Formel (II) angegebene Struktureinheit enthält:

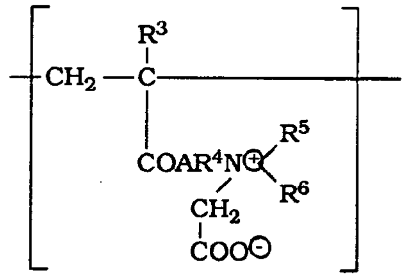

[II]

worin $R^3$ ein Wasserstoffatom oder eine Methylgruppe,
$R^4$ eine Alkylengruppe mit einer Kohlenstoffanzahl von 1 bis 4,
$R^5$ und $R^6$jeweils ein Wasserstoffatom oder eine Alkylgruppe mit einer Kohlenstoffanzahl von 1 bis 4 und A ein Sauerstoffatom oder eine NH-Gruppe bedeuten.

3. Zusammensetzung nach Anspruch 2, wobei das amphotere Polymer 5 bis 80 Gew. -% der Struktureinheit der Formel (I) und 30 bis 90 Gew. -% der Struktureinheit der Formel (II) enthält und ein Durchschnittsmolekulargewicht von 400 bis 2.000.000 besitzt.

4. Zusammensetzung nach Anspruch 1, wobei das anphotere Polymer eine durch die folgende Formel angegebene Struktureinheit:

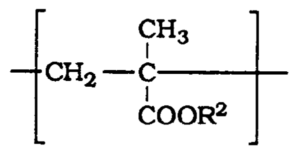

worin $R^2$ eine Alkylgruppe mit einer Kohlenstoffanzahl von 8 bis 20 oder eine Cycloalkylgruppe mit einer Kohlenstoffanzahl von 5 bis 7 bedeutet; sowie eine durch die folgende Formel angegebene Struktureinheit:

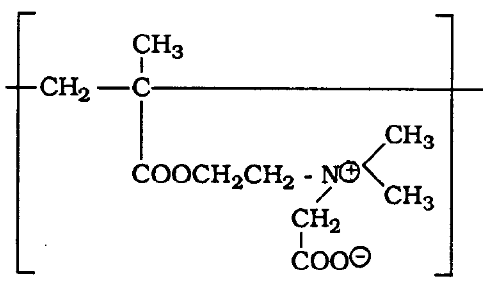

enthält.

5. Zusammensetzung nach Anspruch 1, wobei das amphotere Polymer in einer Menge von 0,001 bis 10 Gew.-% der Zusammensetzung eingemischt ist.

6. Zusammensetzung nach Anspruch 1, wobei ein nichtionisches oberflächenaktives Mittel, das bei -5°C in flüssiger Form oder Pastenform vorliegt und einen HLB-Wert von 10 oder weniger besitzt, hierin eingebracht ist.

7. Zusammensetzung nach Anspruch 6, wobei das nichtionische oberflächenaktive Mittel, das bei -5°C in flüssiger Fonn oder Pastenform vorliegt und einen HLB-Wert von 10 oder weniger besitzt, in einer Menge von 0,1 bis 10 Gew.- % der Zusammensetzung eingebracht ist.

8. Zusammensetzung nach Anspruch 1, wobei die kristalline Verbindung aus aliphatischen Säuren mit einer

Kohlenstoffanzahl von 8 bis 30 und Salzen hiervon, aliphatischen Alkoholen mit einer Kohlenstoffanzahl von 8 bis 30 und aliphatischen Säuredenvaten mit einer Kohlenstoffanzahl von 8 bis 30 gewählt ist.

**9.** Zusammensetzung nach Anspruch 1, wobei die kristalline Verbindung innerhalb eines Bereichs zwischen einer Menge entsprechend der Löslichkeit bei -20°C oder mehr und einer Menge entsprechend der Löslichkeit bei 30°C oder weniger eingebracht ist.

## Revendications

**1.** Composition liquide contenant un composé cristallin dissous dans un solvant, qui comprend au moins un type de polymère amphotère contenant un motif constitutif représenté par la formule (I) suivante :

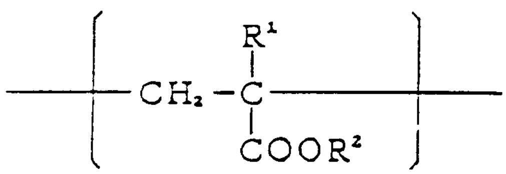

··· [ I ]

dans laquelle $R^1$ est un atome d'hydrogène ou un groupe méthyle, $R^2$ est un groupe alkyle ayant un nombre d'atomes de carbone de 8 à 20 ou un groupe cycloalkyle ayant un nombre d'atomes de carbone de 5 à 7,
formulé dans ladite composition.

**2.** Composition selon la revendication 1, dans laquelle ledit polymère amphotère contient un motif constitutif représenté par ladite formule (I) et un motif constitutif représenté par la formule (II) suivante :

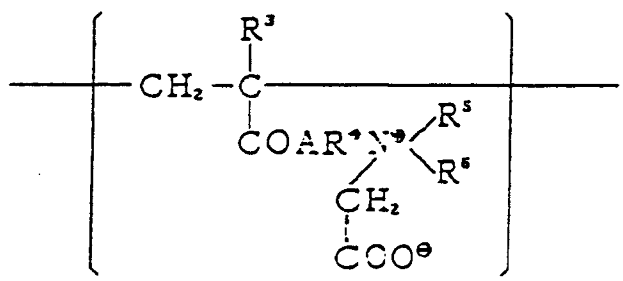

··· [ II ]

dans laquelle $R^3$ est un atome d'hydrogène ou un groupe méthyle, $R^4$ est un groupe alkylène ayant un nombre d'atomes de carbone de 1 à 4, $R^5$ et $R^6$ sont des atomes d'hydrogène ou des groupes alkyle ayant respectivement un nombre d'atomes de carbone de 1 à 4 et A est un atome d'oxygène ou un groupe NH.

**3.** Composition selon la revendication 2, dans laquelle ledit polymère amphotère contient 5 à 80% en poids du motif constitutif de formule (I) et 30 à 90% en poids du motif constitutif de formule (II) et a une masse moléculaire moyenne de 400 à 2 000 000.

**4.** Composition selon la revendication 1, dans laquelle ledit polymère amphotère contient un motif constitutif représenté par la formule suivante :

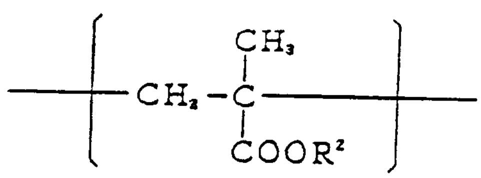

dans laquelle $R^2$ est un groupe alkyle ayant un nombre d'atomes de carbone de 8 à 20 ou un groupe

cycloalkyle ayant un nombre d'atomes de carbone de 5 à 7, et un motif constitutif représenté par la formule suivante :

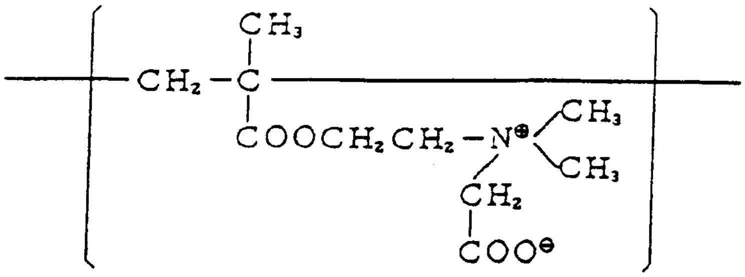

5. Composition selon la revendication 1, dans laquelle ledit polymère amphotère est mélangé à raison de 0,001 à 10% en poids de la composition.

6. Composition selon la revendication 1, dans laquelle est formulé un tensioactif non ionique se présentant sous forme de liquide ou de pâte à -5°C et ayant un rapport hydrolipophile inférieur ou égal à 10.

7. Composition selon la revendication 6, dans laquelle le tensioactif non ionique se présentant sous forme de liquide ou de pâte à -5°C et ayant un rapport hydrolipophile inférieur ou égal à 10 est formulé à raison de 0,1 à 10% en poids de la composition.

8. Composition selon la revendication 1, dans laquelle ledit composé cristallin est choisi parmi les acides aliphatiques ayant un nombre d'atomes de carbone de 8 à 30 et leurs sels, les alcools aliphatiques ayant un nombre d'atomes de carbone de 8 à 30 et les dérivés d'acides aliphatiques ayant un nombre d'atomes de carbone de 8 à 30.

9. Composition selon la revendication 1, dans laquelle ledit composé cristallin est formulé en une quantité comprise entre la quantité correspondant à une solubilité à -20°C ou plus et la quantité correspondant à une solubilité à 30°C ou moins.